# EUROPEAN PATENT APPLICATION

(11) **EP 4 219 701 A1**
(43) Date of publication of application: **02.08.2023**
(21) Application number: 22154185.7
(22) Date of filing: 31.01.2022
(51) Int. Cl.: C12N 9/26, C12Q 1/40

(54) **SENSITIVE ASSAY FOR THE QUANTITATIVE DETERMINATION OF AMYLASE ACTIVITY**

(71) Applicant: Universität Hohenheim, 70599 Stuttgart (DE)
(72) Inventor: FISCHER, Lutz, 73274 Notzingen (DE); REICHENBERGER, Katrin, 73760 Ostfildern (DE); LUTZ-WAHL, Sabine, 73230 Kirchheim (DE)
(74) Representative: Müller-Boré & Partner Patentanwälte PartG mbB

(57) **Abstract**

The present invention relates to methods for the detection of amylase activity in a sample, as well as to kits that can be used in said methods.

## Description

The present invention relates to methods for the detection of amylase activity in a sample, as well as to kits that can be used in said methods.

Amylases are used in many areas, such as in the food industry, e.g., in the production of baked goods or glucose syrups, the textile industry and the detergent industry. Due to the wide application of amylases, there are many areas in which the sensitive and quantitative determination of amylase activities is of great importance. These include, for example, the determination of amylase activities in wheat flours, as this has a major influence on the quality of baked goods. The determination of the residual activities of added amylase preparations in baked bread is also important with regard to a possible declaration obligation.

Many assays for the determination of amylase activity have already been described in the literature; these can be divided into different groups. Amylase activities can be determined, for example, by the increase in reducing sugars. This group of assays includes, for example, the Somogyi-Nelson and the dinitrosalicylic acid assay. In addition, there are assays that measure the decrease in the substrate starch. These include the detection of starch with an iodine-potassium iodide solution or turbidimetric methods. Another possibility for determining amylase activity is the use of chromogenic substrates. These substrates include, for example, starch or amylose substrates labelled with a dye, such as azurine-crosslinked amylase or starch coupled with an azo dye. In addition, the Ceralpha assay (determination of endo-amylase activities) and the Betamyl3 assay (determination of exo-amylase activities) also belong to this group. Here, p-nitrophenyl-maltoheptaoside (BPNPG7) (Ceralpha) and *p-*nitrophenyl-beta-D-maltotrioside (PNPbeta-G3) (Betamyl3) are used as substrate. The substrate is then first cleaved by an endo/exo amylase. The p-nitrophenyl maltosaccharide (Ceralpha) or p-nitrophenyl-beta-D-glucose (Betamyl3) released in the first reaction are then immediately degraded by an excess of amyloglucosidase and alpha-glucosidase (Ceralpha) or beta-glucosidase (Betamyl3). This releases p-nitrophenol, which is then quantified photometrically.

With the commercially available kits known in the art, amylase activities can be reliably determined up to an enzyme activity of 0.05 U/ml (equivalent to 0.84 nkat/mL) in, for example, foodstuffs. However, the sensitivity of these assays is not sufficient for certain problems, for example for the determination of the residual activity of certain amylases in bread. Thus, the sensitivity of previous assays for the determination of amylase activities is low. Moreover, some of the existing assays involve many and sometimes complex steps (e.g., heating at 95 °C). Further, some known assays use synthetic substrates, which can be disadvantageous for the calculation of required activity amounts.

Accordingly, the technical problem underlying the present invention is the provision of means for the detection of amylase activity that are most importantly characterized by a superior detection sensitivity. Further, such means should allow for a time-, labor- and cost-efficient detection of amylase activity and should be able to use natural amylase substrates.

The solution to the above technical problem is achieved by the embodiments characterized in the claims.

In particular, in a first aspect, the present invention relates to a method for the detection of amylase activity in a sample, comprising the steps of
(a) adding a substrate to the sample, wherein said substrate is selected from the group consisting of starch, glycogen, amylose, amylopectin, maltotriose, maltotetraose, and combinations thereof,
(b) incubating the sample at conditions and for a time allowing for the enzymatic conversion of the substrate into glucose and maltose by amylases that might be present in the sample,
(c) adding to the sample incubated in step (b)
   (i) glucose oxidase,
   (ii) peroxidase, and
   (iii) DA-67 (sodium;2-[[3,7-bis(dimethylamino)phenothiazine-10-carbonyl]amino]acetate),
(d) incubating the sample at conditions and for a time allowing for
   (i) the enzymatic conversion of glucose and maltose that might have been generated in step (b) into oxidized glucose, oxidized maltose, and hydrogen peroxide (H₂O₂) by said glucose oxidase, and
   (ii) the enzymatic conversion of DA-67 and H₂O₂ that might have been generated in step (d)(i) into reduced DA-67 (methylene blue; [7-(dimethylamino)phenothiazin-3-ylidene]-dimethyl-azanium;chloride) by said peroxidase, and
(e) detecting methylene blue in the sample.

As used herein, the term "detection of amylase activity" relates to qualitative detection, i.e., detection of the presence or absence of any amylase activity, or to quantitative detection, *i.e.,* detection of a specific amount of amylase activity, wherein quantitative detection is preferred.

Further, the term "amylase activity" as used herein relates to the capability of a given amylase to catalyze the hydrolysis of starch, glycogen, amylose, amylopectin, maltotriose, and maltotetraose into sugars in a random manner.

Amylases of which the activity can be detected with the methods of the present invention are not particularly limited and include any alpha-amylases, any beta-amylases, any maltogenic amylases, and any alpha-glucosidases, as known in the art.

According to the present invention, the substrate added in step (a) of the above methods is selected from the group consisting of starch, glycogen, amylose, amylopectin, maltotriose, maltotetraose, and combinations thereof. In preferred embodiments, the substrate is starch.

The sample to be analyzed in the methods of the present invention can be any sample, preferably any aqueous sample, being suspected of containing one or more amylases. Means for preparing suitable samples are not particularly limited and are known in the art.

In step (a) of the methods of the present invention, the substrate is added to the sample, preferably in the form of an aqueous solution. Suitable buffers and means for preparing an aqueous substrate solution are not particularly limited and are known in the art. By way of example, the substrate can be solubilized in a buffer comprising 50 mM 2-(N-morpholino)ethanesulfonic acid (MES), 2 mM CaCl₂, 1 g·L⁻¹ Na-azide, and 1 g·L⁻¹ bovine serum albumin (BSA). Respective aqueous substrate solutions preferably comprise the substrate in a concentration of from about 0.005 to about 0.015 % (w/v), preferably from about 0.006 to about 0.014 % (w/v), more preferably from about 0.007 to about 0.013 % (w/v), more preferably from about 0.008 to about 0.012 % (w/v), more preferably from about 0.009 to about 0.011 % (w/v), and most preferably a concentration of about 0.001 % (/w/v).

In step (b) of the methods of the present invention, the sample obtained after the addition of the substrate in step (a) is incubated at conditions and for a time allowing for the enzymatic conversion of the substrate present in the sample into glucose and maltose by one or more types of amylases that might be present in the sample. Suitable conditions in this respect include incubation at about 20 to about 60 °C, more preferably at about 30 to 50 °C, more preferably at about 37 to about 43 °C, more preferably at about 38 to about 42 °C, more preferably at about 39 to about 41 °C, most preferably at about 40°C, and/or incubation for a duration of about 20 min to about 90 min (1.5 h), preferably of about 30 min to about 90 min, more preferably of about 40 min to about 80 min, more preferably of about 50 min to about 70 min, and most preferably for a duration of about 60 min (1 h). In specific embodiments, the aqueous substrate solution can be preincubated prior to step (a) of the methods of the present invention to bring said aqueous substrate solution to the temperature that will be used for incubation in step (b).

In step (c) of the methods of the present invention, glucose oxidase (e.g., *Aspergillus niger* glucose oxidase), peroxidase (e.g., horseradish peroxidase), and DA-67 (sodium;2-[[3,7-bis(dimethylamino)phenothiazine-10-carbonyl]-amino]acetate) are added to the sample incubated in step (b). These components can be mixed together prior to adding the resulting mixture to the sample.

In specific embodiments, glucose oxidase is added to the sample in an amount of about 10 to about 200 U·ml⁻¹ in the sample, i.e., the glucose oxidase is added to the sample in an amount resulting in the presence of about 10 to about 200 U·ml⁻¹ in the sample after adding the same to the sample. Preferably, glucose oxidase is added to the sample in an amount of about 20 to about 180 U·ml⁻¹ in the sample, more preferably of about 30 to about 170 U·ml⁻¹ in the sample, more preferably of about 40 to about 160 U·ml⁻¹ in the sample, more preferably of about 50 to about 150 U·ml⁻¹ in the sample, more preferably of about 60 to about 140 U·ml⁻¹ in the sample, more preferably of about 70 to about 130 U·ml⁻¹ in the sample, more preferably of about 80 to about 120 U·ml⁻¹ in the sample, more preferably of about 80 to about 110 U·ml⁻¹ in the sample, more preferably of about 85 to about 105 U·ml⁻¹ in the sample, more preferably of about 90 to about 100 U·ml⁻¹ in the sample, more preferably of about 90 to about 95 U·ml⁻¹ in the sample, more preferably of about 91 to about 95 U·ml⁻¹ in the sample, more preferably of about 92 to about 94 U·ml⁻¹ in the sample, and most preferably in an amount of about 93 U·ml⁻¹ in the sample.

In further specific embodiments, peroxidase is added to the sample in an amount of about 1 to about 5 U·ml⁻¹ in the sample, i.e., the peroxidase is added to the sample in an amount resulting in the presence of about 1 to about 5 U·ml⁻¹ in the sample after adding the same to the sample. Preferably, peroxidase is added to the sample in an amount of about 1.5 to about 4.5 U·ml⁻¹ in the sample, more preferably of about 2 to about 4 U·ml⁻¹ in the sample, more preferably of about 2.2 to about 3.8 U·ml⁻¹ in the sample, more preferably of about 2.4 to about 3.6 U·ml⁻¹ in the sample, more preferably of about 2.6 to about 3.4 U·ml⁻¹ in the sample, more preferably of about 2.8 to about 3.2 U·ml⁻¹ in the sample, more preferably of about 2.9 to about 3.1 U·ml⁻¹ in the sample, and most preferably in an amount of about 3 U·ml⁻¹ in the sample.

In yet further specific embodiments, DA-67 is added to the sample in an amount of about 15 to about 100 µM in the sample, i.e., in an amount resulting in a DA-67 concentration of about 15 to about 100 µM in the sample after adding the same to the sample. Preferably, DA-67 is added to the sample in an amount of about 20 to about 80 µM in the sample, more preferably of about 30 to about 70 µM in the sample, more preferably of about 35 to about 65 µM in the sample, more preferably of about 40 to about 60 µM in the sample, more preferably of about 45 to about 55 µM in the sample, and most preferably in an amount of about 50 µM in the sample.

In step (d) of the methods of the present invention, the sample obtained after the addition of glucose oxidase, peroxidase, and DA-67 in step (c) is incubated at conditions and for a time allowing for (i) the enzymatic conversion of glucose and maltose that might have been generated in step (b) into oxidized glucose, oxidized maltose, and hydrogen peroxide (H₂O₂) by said glucose oxidase, and (ii) the enzymatic conversion of DA-67 and H₂O₂ that might have been generated in step (d)(i) into reduced DA-67 (methylene blue; [7-(dimethylamino)phenothiazin-3-ylidene]-dimethyl-azanium;chloride) by said peroxidase. Suitable conditions in this respect include incubation at about 35 to about 40 °C, preferably at about 36 to 40 °C, more preferably at about 37 to about 40 °C, more preferably at about 38 to about 40 °C, more preferably at about 39 to about 40 °C, most preferably at about 40°C, and/or incubation for a duration of about 0.5 to about 11.5 h, preferably of about 1 to about 11 h, more preferably of about 2 to about 11 h, more preferably of about 3 to about 11 h, more preferably of about 4 to about 11 h, more preferably of about 5 to about 10 h, more preferably of about 6 to about 9 h, more preferably of about 6.5 to about 8.5 h, more preferably of about 7 to about 8 h, and most preferably for a duration of about 7.5 h. In specific embodiments, a mixture of glucose oxidase, peroxidase, and DA-67 can be preincubated prior to step (c) of the methods of the present invention to bring said mixture to the temperature that will be used for incubation in step (d).

In step (e) of the methods of the present invention, methylene blue is detected in the sample after incubation in step (d). Means for detecting methylene blue in a sample are not particularly limited and are known in the art. In specific embodiments, detection of methylene blue is photometric detection at a wavelength of 620 nm, as known in the art.

According to the methods of the present invention, amylase activity is detected in case methylene blue is detected in step (e) of said methods. As indicated above, such detection is preferably quantitative detection, i.e., detection includes the determination of a specific amount of amylase activity. In such cases, step (e) of the methods of the present invention comprises the steps of
(e1) detecting methylene blue in the sample,
(e2) comparing the amount of methylene blue detected in step (e1) to the amounts of methylene blue in a calibration curve correlating known amylase activities to said amounts of methylene blue, and
(e3) determining the amylase activity corresponding to the amount of methylene blue detected in step (e1) using said calibration curve;
   or step (e) comprises the steps of
(e1') kinetically detecting methylene blue in the sample over time,
(e2') determining the slope of methylene detection over time, and
(e3') determining the difference in the slope determined in step (e2') to a reference, and
(e4') determining the amylase activity on the basis of the difference determined in step (e3').

Respective means of establishing a suitable calibration curve and using said calibration curve to determine the amylase activity corresponding to the amount of methylene blue detected in step (e1) are not particularly limited and are known in the art. Further, respective means of kinetically detecting methylene blue in the sample over time, determining the slope of methylene detection over time, determining the difference in the slope determined in step (e2') to a reference (e.g., the slope determined for a heat-inactivated sample or buffer), and determining the amylase activity on the basis of the difference determined in step (e3') are not particularly limited and are known in the art.

The methods of the present invention can be performed in any suitable kind of reaction vessel, e.g., in multiwell microtiter plates, such as 96-well microtiter plates, wherein a multitude of parallel assays can be performed on such plates in the multitude of wells.

In a second aspect, the present invention relates to a kit, comprising
(a) a substrate, wherein said substrate is selected from the group consisting of starch, glycogen, amylose, amylopectin, maltotriose, maltotetraose, and combinations thereof,
(b) glucose oxidase,
(c) peroxidase, and
(d) DA-67 (sodium;2-[[3,7-bis(dimethylamino)phenothiazine-10-carbonyl]-amino]acetate).

In this aspect, the substrate, glucose oxidase and peroxidase are as defined above for the first aspect of the present invention.

In specific embodiments, the kits of the present invention can further comprise suitable buffers or buffer components, e.g., 2-(N-morpholino)ethanesulfonic acid (MES), CaCl₂, and/or bovine serum albumin (BSA). Further, the kits of the present invention can further comprise glucose, maltose and/or a specific amylase for the purpose of establishing calibration curves.

As used herein, the term "comprising"/"comprises" expressly includes the terms "consisting essentially of"/"consists essentially of" and "consisting of"/"consists of", *i.e.,* all of said terms are interchangeable with each other herein.

Further, as used herein, the term "about" preferably represents a modifier of the specified value of ± 10%, more preferably ± 10%, ± 8%, ± 6%, ± 5%, ± 4%, ± 3%, ± 2%, ± 1%, or ± 0.5%. Thus, by way of example, the term "about 100" can include the ranges of 90 to 110, 92 to 108, 94 to 106, 95 to 105, 96 to 104, 97 to 103, 98 to 102, 99 to 101, or 99.5 to 100.5.

The present invention relates to a new assay for the determination of amylase activity whose sensitivity is at least a factor of 170 higher than the sensitivity of commercially available assays known on the art. The higher sensitivity of the assay of the present invention, thus, makes it possible to e.g., determine the residual activity of an alpha-amylase from *Bacillus subtilis* in white bread. The determination of this residual activity was not possible with the commercially available assays for the determination of endo-amylase activities.

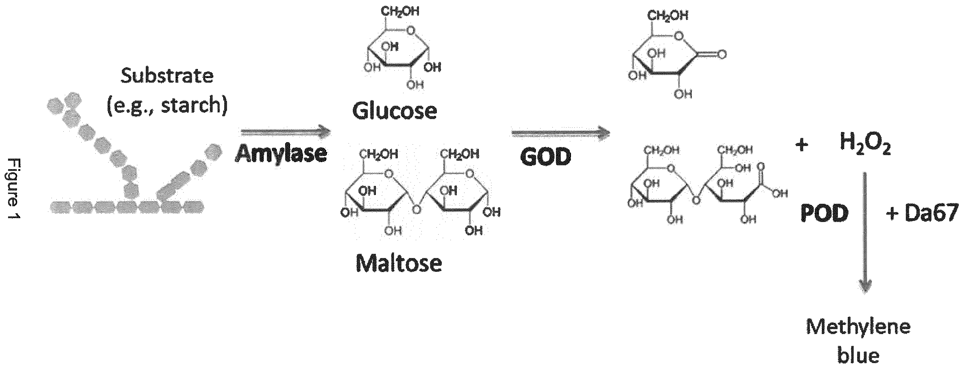

The newly developed assay is based on the conversion of a natural substrate, selected from the group consisting of starch, glycogen, amylose, amylopectin, maltotriose, maltotetraose, and combinations thereof, by the amylase and the use of two auxiliary enzymes. The principle of the assay is shown in Figure 1.

The saccharides glucose and maltose released during the hydrolysis of the substrate can be oxidized with oxygen using the auxiliary enzyme glucose oxidase (GOD, e.g., from *Aspergillus niger).* The resulting hydrogen peroxide is then reduced by a peroxidase (POD, e.g., from horseradish), transferring electrons to an electron acceptor (DA-67; (sodium;2-[[3,7-bis(dimethylamino)phenothiazine-10-carbonyl]amino]acetate). The amount of reduced DA-67 is then quantified, e.g., photometrically at a wavelength of 620 nm.

After optimization of the concentrations of the assay components (DA-67, peroxidase, starch), a limit of detection (LOD) of 0.25 pkat·mL⁻¹ and a limit of quantification (LOQ) of 0.33 pkat·mL⁻¹ could be achieved with an assay run time of 7.5 h. This corresponds to a maltose concentration of 4.5 pM (LOD) and 6.1 pM (LOQ). Specifically, the optimized concentrations were 50 µM of the dye (DA-67), 3 U·mL⁻¹ peroxidase, 93 U·mL⁻¹ glucose oxidase and 0.01 % (w/v) starch.

The developed assay was evaluated with two amylases, a maltogenic amylase from *Geobacillus stearothermophilus* and an alpha-amylase from *Bacillus subtilis.* With the enzymes tested, a minimum preparation concentration of 3.2 ng·mL⁻¹ of the maltogenic amylase and 3.5 ng·mL⁻¹ of the alpha-amylase was detected. Compared to the commercially available kits for the determination of endo-amylase activities (Ceralpha kit) and exo-amylase activities (Betamyl3 kit), this corresponds to an increase in sensitivity by a factor of 171 (maltogenic amylase) and 1561 (alpha-amylase) with an assay time of 20 min and the use of MES buffer (50 mM, pH 5.5). Comparing the commercial assays with the new assay of the present invention with the maximum run time of 7.5 h, this factor increases to 785 (maltogenic amylase) and 7160 (alpha-amylase).

Amylase activity assays using the enzymatic detection of the cleavage products when using the natural substrate starch have already been described, and the principle of determining the maltose released by amylase activity using the two auxiliary enzymes glucose oxidase and peroxidase has already been used. In a respective study, the inhibitory effects of different molecules on alpha-amylase were determined using a glucose content determination kit. In this kit, phenol and 4-aminoantipyrene were used as electron acceptors. The reduction produced a quinone which was photometrically quantified at 505 nm. In this study, a limit of detection (LOD) of 0.69 mM maltose was achieved. This makes the newly developed assay of the present invention 153 times more sensitive.

The newly developed assay of the present invention for the determination of amylase activities is more sensitive than the commercially available assays (Ceralpha/Betamyl3 kits) and uses a natural substrate, selected from the group consisting of starch, glycogen, amylose, amylopectin, maltotriose, maltotetraose, and combinations thereof. This allows for the determination of very low amylase activities, which was not possible until now. The new assay can be performed kinetically in microtiter plates (96 preparations). This allows a large sample turnover with relatively little work.

The use of a natural substrate is advantageous in certain cases, for example to estimate the activity required for an application. The commercially available kits (Ceralpha and Betamyl3) use other, synthesized substrates. The activity towards these substrates differs from the activity of the amylases towards the natural substrate. Therefore, the determined activities cannot be used directly to estimate an activity for an application.

The cost of the newly developed assay compared to the commercially available assays (Ceralpha/Betamyl3) is significantly lower. The newly developed assay costs 3.4 cents per approach. In contrast, one preparation of the Ceralpha or Betamyl3 assay costs 1.95 and 1.93 euros, respectively.

The figures show:
Figure 1:
   Principle of the new amylase assay (GOD: glucose oxidase, POD: peroxidase,
   Da67: sodium;2-[[3,7-bis(dimethylamino)phenothiazine-10-carbonyl]amino]-acetate (DA-67).
Figure 2:
   Influence of the starch content on the difference of the slope of the maltose sample (20 *µ*M) and the blank.
Figure 3:
   Evaluation of the new amylase assay using the alpha-amylase from *B. subtilis* (upper panel) and the maltogenic amylase from G. *stearothermophilus* (lower panel). The kinetic measurement was done at 40 °C in triplicate.
Figure 4:
   The limit of quantification (LOQ) of the new amylase assay with varying amylase action times.

The present invention will be further illustrated by the following example without being limited thereto.

### Examples

### Materials and methods:

### Chemicals and materials

All chemicals were of analytical grade and obtained from Sigma-Aldrich (Taufkirchen, Germany), Carl Roth GmbH (Karlsruhe, Germany), AppliChem (Darmstadt, Germany), and Serva (Heidelberg, Germany). Sodium;2-[[3,7-bis(dimethylamino)phenothiazine-10-carbonyl]amino]acetate (DA-67) was purchased from Fujifilm Wako Pure Chemical Corporation (Osaka, Japan). The glucose oxidase from *Aspergillus niger* was purchased from Roth (product number 6028.1), the peroxidase from horse radish practical grade I was purchased from Aplichem (Darmstadt, Germany). Wheat flour type 550 was purchased from Rettenmeier Mühle (batch number: 70004599, expiration date: 13.02.2021). Commercial dry yeast was purchased from Saf-instant (LeSaffre, Buhl, Germany) and hydrogenated peanut fat from BÄKO (Ladenburg, Germany). All buffers and solutions were prepared using double-distilled and autoclaved water. All reaction tubes, glass vessels, spoons, and other similar objects were autoclaved before use to minimize the risk of amylase contamination. All working steps were conducted wearing gloves.

### Statistical analysis

All baking experiments, extractions, and activity determinations were carried out in triplicate. Standard deviations were calculated using Excel (Microsoft, Redmond, USA). The slopes of the kinetic measurements and the coefficient of determination (R²) were also determined with Excel. The limit of detection (LOD) and limit of quantification (LOQ) were evaluated according to DIN ISO 5725.2 and DIN 32646, respectively, as known in the art. Thus, the LOD of the assay is defined as six times the standard deviation of the reference values. The LOQ is defined as nine times the standard deviation of the reference values. For these determinations at least 12 reference values were measured.

### Amylase preparations

Two powdered food-grade and food-approved amylase preparations were used for the evaluation of the new assay. First, the maltogenic amylase from *Geobacillus (G.) stearothermophilus* and second, an alpha-amylase from *Bacillus (B.) subtilis.* The alpha-amylase from *B. subtilis* was also used for the baking experiments. White bread was made with three different enzyme dosages covering the recommendation from the manufacturer (150 - 650 µg enzyme preparation per g flour, 150 - 650 ppm regarding flour weight). The amylase was added at 150, 375 and 650 µg per g flour. In order to determine the specific activity of the amylase preparations used, the powder was suspended in standard assay buffer (50 mM 2-(N-morpholino)ethansulfonic acid (MES), 2 mM CaCl₂, 0.1 % (w/v) Na-azide, pH 5.5) with 1.5 g bovine serum albumin (BSA) per liter. The suspension was prepared with an amylase preparation concentration of 15 g per liter and shaken for 45 min at 20 °C. Then, the suspension was centrifuged at 6800 g for 10 min at 4 °C. The supernatant was loaded on PD10 columns and eluted using 50 mM MES, 2 mM CaCl₂ and 0.1 % (w/v) Na-azide, pH 5.5. The final concentration of the amylase preparation was 10 g·L⁻¹ with a concentration of 1.0 g BSA per liter due to dilution during the buffer change with PD10 columns.

### Procedure and optimization of the new amylase assay

The new amylase assay was measured kinetically with a measurement interval of 30 s for 30 min. The assay was performed in microtiter plates (96 well) at 40 °C. The slope between 0.5 and 1 h was used for the calculation of the released sugars. It was determined by linear regression with a coefficient of determination (R²) of at least 0.999. One reaction approach comprised of 200 µL sample, 30 µL of Da67 solution, 50 µL peroxidase solution, 10 µL starch solution and 10 µL glucose oxidase (GOD) solution. The stock solutions of the different components were all prepared in standard buffer (50 mM MES, 2 mM CaCl₂, 1 g·L⁻¹ Na-azide, 1 g·L⁻¹ BSA) and stored separately. The peroxidase, glucose oxidase and DA-67 solutions were stored on ice. The DA-67 solution was additionally stored in the dark. The sample solution (200 µL) was preincubated for 15 min at 40 °C in a photometer. The other assay components were combined directly before use to a "reaction mixture" and preincubated also for 15 min in a thermoshaker. The reaction mixture (100 µL) was then added to the sample and the kinetic measurement was started after 30 min. As reference a sample with buffer instead of sample was used. For an assay run time of 0.5 h the reaction mixture was added directly after the incubation time and kinetic measurement was started after 0.5 h. For longer assay run times the assay was done in two steps, in the first step the sample solution containing the amylase was incubated together with the starch solution at 40 °C in sealed microtiter plates in a thermoshaker. After the incubation time the reaction cocktail was added, and the kinetic measurement of the absorption was started after another 0.5 h. Calibration curves were prepared using 0.78 to 12.5 µM glucose and 0.78 to 50 µM maltose with starch in the assay approach. The activities were calculated with maltose as standard.

For the optimization of the assay, a DA-67 concentration of 50 µM, a glucose oxidase concentration of 93 U·mL⁻¹, a starch concentration of 0.1 % (w/v) and a peroxidase activity of 3 U·mL⁻¹ was used. One unit of glucose oxidase is defined as the amount of enzyme that catalyzed the oxidation of 1 µM glucose per minute at pH 7.0 and 25 °C. One unit of peroxidase is defined as the amount of enzyme that forms 1.0 mg pupurogallin from pyrogallol in 20 s at pH 6.0 and 20 °C. To find optimal concentrations for the different components, their concentration was varied one after the other. The optimized concentration of one assay component was then used for the next optimization step. The concentration of DA-67 was varied between 12.5 and 200 µM, the concentration of peroxidase was varied between 0.75 and 12 U·mL⁻¹, the concentration of glucose oxidase was varied between 9.3 and 466.6 U·mL⁻¹ and the concentration of starch was varied between 0.01 and 0.2 % (w/v). All given concentrations herein are referred to the concentrations in the assay and not the stock solutions.

For the determination of the residual activity in white bread, the freeze-dried and ground bread (75 mg) was resuspended in buffer containing 0.01 % (w/v) starch (50 mM MES, 2 mM CaCl₂, 1 g·L⁻¹ Na-azide, 1 g·L⁻¹ BSA; 1.2 mL). The samples were centrifuged and 100 µl of the supernatant was removed. The supernatant (1) was used for the determination of maltose content in the bread before the incubation step. The samples were vortexed carefully to resuspend the bread particles and incubated on a rotary shaker with 300 rpm at 40 °C. After incubation, the bread particles were removed by centrifugation. The supernatant (2) was used for the determination of the maltose content of the samples at the end. Supernatant (1) and (2) were diluted by the factor of 100 and used in the optimized new amylase assay. For the calculation of the activity, the difference of the starting and end concentrations was used. The difference from the reference bread was subtracted from the difference of the amylase supplemented bread.

### Determination of amylase activities with Ceralpha and Betamyl3 assay according to the data sheet

The activity of the alpha-amylase from *B. subtilis* was investigated using the endo-amylase Ceralpha (HR) reagent (product code: R-AMHR4) from Megazyme (Wicklow, Ireland). The Ceralpha reagent contained non-reducing-end blocked p-nitrophenyl maltoheptaoside (BPNG7) and an excess of a thermostable alpha-glucosidase, which has no action on BPNG7. Amylases with an endo-acting activity are able to cleave the substrate, the released p-nitrophenyl maltosaccharide fragment is immediately hydrolyzed into glucose and p-nitrophenol by the alpha-glucosidase. The activity of the maltogenic amylase from *G. stearothermophilus* was investigated using the Betamyl3 reagent (product code: R-BAMR3) from Megazyme (Wicklow, Ireland). The Betamyl3 reagent contained p-nitrophenyl-beta-D-maltotrioside (PNP-G3) and an excess of beta-glucosidase. An exo-acting amylase hydrolyzes the substrate to maltose and p-nitrophenyl-beta-D-glucose. The p-nitrophenyl-beta-D-glucose is immediately hydrolyzed into D-glucose and p-nitrophenol by the beta-glucosidase. The released p-nitrophenol is then determined at 400 nm.

According to the instructions for the Ceralpha and Betamyl3 assay, 0.2 mL of preincubated sample (5 min, 40 °C) were mixed with 0.2 mL preincubated Ceralpha/Betamyl3 reagent. After 10 min (Ceralpha) or 20 min (Betamyl3), 3 mL of the stopping reagent (Ceralpha assay: 1 % (w/v) tri-sodium phosphate L⁻¹, pH 11.0, Betamyl3 assay: 1 % Tris buffer, pH 8.5) were added. The absorbance was measured at a wavelength of 400 nm. As reference 1 stopping reagent was added to Ceralpha/Betamyl3 reagent, followed by the addition of sample solution. Calibration curves were done with p-nitrophenol with 0.05 to 3.2 mM for the Ceralpha assay and 0.006 to 0.1 mM for the Betamyl3 assay.

In addition, the activity of the maltogenic amylase from G. *stearothermophilus* was investigated with an optimized version of the Betamyl3 assay, as known in the art, and the Ceralpha assay. For the optimization of the Ceralpha assay 50 µL preincubated amylase (3 min, 40 °C) were mixed with 50 µL preincubated Ceralpha HR reagent (3 min, 40 °C). Once the solution turned yellow, the reaction was stopped with 375 µL of 2 % (w/v) Tris, pH 11.0. 240 µL were transferred to a microtiterplate and measured at 405 nm. A calibration curve with p-nitrophenol with concentrations between 0.003 and 0.2 mM was done. For the evaluation of the assay a second reference was tested. Therefore, Ceralpha HR reagent was incubated with standard assay buffer (50 mM MES, 2 mM CaCl₂, 1 g·L⁻¹ BSA, 0.1 % (w/v) Na-azide) for the respective assay run time followed by the addition of the stopping reagent (reference 2). For the determination of the minimal detectable (LOD) and minimal quantifiable (LOQ) volumetric activity, 12 references 2 were made for an assay run time of 24 h. To check if long assay run times lead to reliable results a solution of the alpha-amylase from *B subtilis* was diluted in standard assay buffer to volumetric activities between 0.5 and 60.0 pkat·mL⁻¹. The thermal stability of the alpha-amylase from *B. subtilis* and the maltogenic amylase from G. *stearothermohpilus* were investigated in standard assay buffer at 90 °C using the optimized Ceralpha and Betamyl3 assays.

### Baking of white bread and determination of the residual amylase activity

The white bread consists of wheat flour type 550 (450 g), dry yeast (4.5 g), sodium chloride (6.75 g), sucrose (4.5 g), fat (4.5 g), and water (300 g). The alpha-amylase from *B. subtilis* was added together with the water to the dough ingredients. Three different dosages between 150 and 650 µg·g_{flour}⁻¹ (150 to 650 ppm) were used. The ingredients were mixed in a spiral mixer (Hobart kneader) for 2 min at level 1 and 5 min at level 2. The primary fermentation step was done for 20 min at 31 °C with 75 to 85 % relative humidity in a proofing cabinet. The next step was a dough relaxation phase of 10 min at room temperature. The dough was shaped, placed in the baking pin, and proofed for 60 min at 31 °C and 75 to 85 % relative humidity in a proofing cabinet. The baking was performed at 230 °C for 35 min with 8 s vapor. For sample taking bread slices from the middle part of the bread were used. For activity measurements only the bread crumb was used. The samples were directly frozen at -80 °C, freeze-dried and ground using a conical drill. The extraction of the amylase from bread samples was optimized using freeze dried, ground bread and different additives in the standard buffer (50 mM MES, 2 mM CaCl₂, pH 5.5) with an extraction time of one hour at 20 °C and 100 rpm on an orbital shaker. The best result of about 80 % recovery compared to the added amylase activity was achieved with 30 % (w/v) maltose in the extraction buffer. Therefore, this extraction procedure was used for the activity measurement using the Ceralpha assay. Bread extracts were loaded on PD10 columns before applied in the Ceralpha assay and were eluted in standard buffer to get rid of the sugars and other residues from the bread.

### Example 1:

### General procedure

A new sensitive amylase assay using starch instead of a synthetic substrate was developed. The new assay can be used for the determination of endo- and exo-amylase activities, respectively, and is based on two ancillary enzymes. The principle of the new amylase assay is shown in Figure 1.

Sugars, like glucose and maltose, which are released through the action of several amylases, such as alpha-, beta- or maltogenic amylases were oxidized by the glucose oxidase from *Aspergillus niger.* In this reaction hydrogen peroxide is formed. Electrons from hydrogen peroxide were transferred to sodium;2-[[3,7-bis(dimethylamino)phenothiazine-10-carbonyl]amino]acetate (DA-67). This reaction is catalyzed by the horseradish peroxidase. The reduced form of the DA-67 (methylene blue; [7-(dimethylamino)phenothiazin-3-ylidene]-dimethyl-azanium;chloride; also known as methylene blue), was quantified photometrically at a wavelength of 620 nm. The new assay was no end-point measurement but a kinetic one and was done at 40 °C. That means that the increase of the absorption is followed over time (every 30 s for 30 min) and the resulting slope of the curves is used for the further assay evaluation. Also, the calibration of this assay was done kinetically, resulting in calibration curves where the slopes are plotted over the sugar concentrations. To achieve a high sensitivity, the concentrations of the different assay components were optimized in a first step. Therefore, the concentrations of the different components were evaluated each after another, where all other components were kept constant.

### Example 2:

### Peroxidase activity and DA-67 concentration

The concentrations of these two components were optimized using a 20 µM maltose sample. An assay for the determination of diamine oxidase activities was used as starting point for the concentrations of the peroxidase and DA-67, as known in the art. The concentrations of the peroxidase and DA-67 were varied from 0.75 to 12 U·mL⁻¹ and from 12.5 to 200 µM, respectively, whereas the other assay components were held constant. No changes were observed in the slope of the maltose sample and the reference (data not shown). Therefore, concentrations as known in the art were used for the further experiments. The used peroxidase was present in excess in the tested range as the slope of the maltose sample was independent from the concentration. This means that all released hydrogen peroxide is immediately consumed by the peroxidase. Therefore, this step is not rate limiting for the assay.

### Example 3:

### Glucose oxidase activity

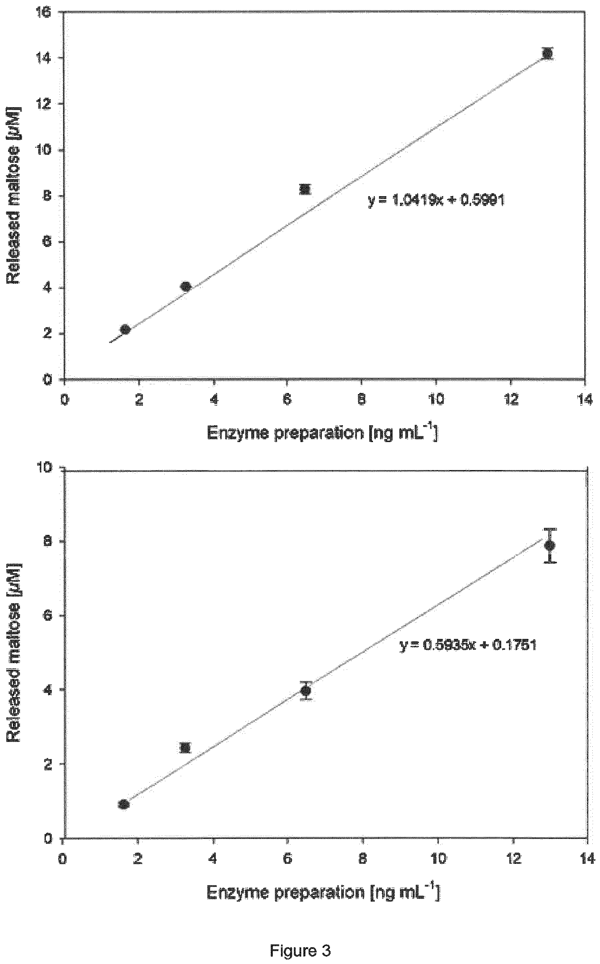

To find a suitable level of glucose oxidase activity, also a 20 µM maltose sample was used. The glucose oxidase activity was varied between 9.3 and 466.6 U·mL⁻¹ in the assay. The correlation of the applied activities and the slope is shown in Figure 3.

The correlation between glucose oxidase activity and the slope of the absorptions was linear and increased from 1.8 min⁻¹ to 2.4 min⁻¹, this corresponds to an increase of 34.4 %.

### Example 4:

### Starch concentration

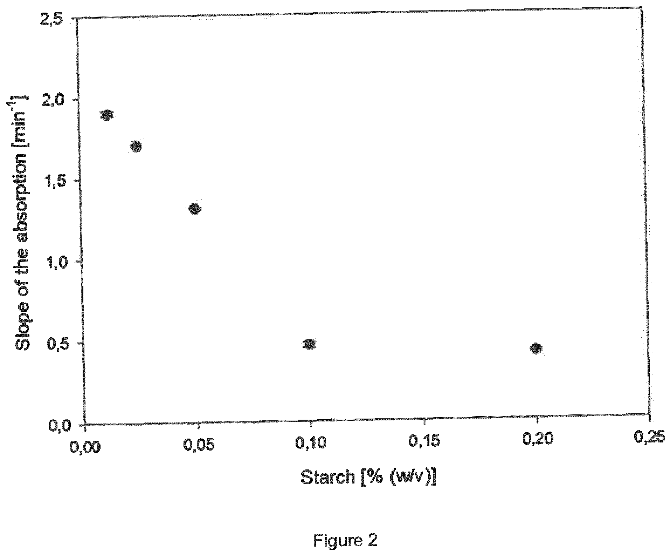

The starch concentration in the assay was varied between 0.01 and 0.2 % (w/v), whereas the concentrations of the other components were kept constant. As a reference, the same sample without GOD was used. The slopes of the reference and the maltose sample were increasing with increasing starch concentration. The differences between the slopes (change of the absorption over time) of the maltose samples and the references are shown in Figure 2.

The difference of the slopes of the maltose sample and the reference decreased with increasing starch concentration. Therefore, it was decided to use the lowest starch concentration because this led to the highest sensitivity/lowest LOD value. To achieve a high sensitivity, the reference values have to be low and the difference between sample and reference have to be high. Therefore, it was decided to use the lowest starch concentration of 0.01 % (w/v). The further experiments were done using a DA-67 concentration of 50 µM, 3 U·mL⁻¹ peroxidase, 93 U·mL⁻¹ glucose oxidase and 0.01 % (w/v) starch.

### Example 5:

### Evaluation of the new assay - Calibration curves with glucose and maltose

Calibration curves with glucose (0.78 - 12.5 µM) and maltose (0.78 - 50 µM) were prepared (data not shown) to check the linearity of the new amylase assay. The slope of the calibration curve with glucose was about 4 times higher than the slope of the calibration curve with maltose (data not shown). This factor of 4 is in correspondence to the findings in the art, where a four times higher activity of the glucose oxidase towards glucose than maltose was also observed. This is logical as the used enzyme is categorized as a glucose and not a maltose oxidase. A previous study used maltose as standard for the amylase activity determinations. This was also done in the present study. The calibration of the new assay with maltose is arbitrary and could also be done with glucose. Also, the achieved activities could easily be transferred to glucose-based activities by just using the glucose calibration curve. The LOD (limit of detection) for the new assay was 1.38 µM maltose. This is about 210 times lower than the LOD value of 290 µM for the assay known in the art.

### Example 6:

### Evaluation of the new assay - Determination of endo- and exo-amylase activities

An alpha-amylase from *B. subtilis* (endo-acting) and a maltogenic amylase from *G. stearothermophilus* (exo-acting) were used for the evaluation of the new assay. Both commercially available amylase preparations were diluted to concentrations of 25 to 100 ngₚᵣₑₚₐᵣₐₜᵢₒₙ·mL⁻¹. The assay was done with a run time of 0.5 h. For both amylases a linear correlation between slope (absorbance increase over time) and concentration of the preparation was found (Figure 3).

The LOQ (limit of quantification) for this assay run time of 0.5 h was 2.3 pkat mL⁻¹. This corresponds to the concentrations of 21.8 ngₚᵣₑₚₐᵣₐₜᵢₒₙ·mL⁻¹ for the alpha-amylase and a 19.1 ng_{preparation·}mL⁻¹ for the maltogenic amylase, respectively.

### Example 7:

### Evaluation of the new assay - Evaluation of the assay run time for amylases

If only little amylase activities would be present in a hypothetical sample, the amount of released sugars is very little with a run time of 0.5 h. Thus, it was evaluated if the assay run time could be prolonged to achieve higher sugar concentrations.

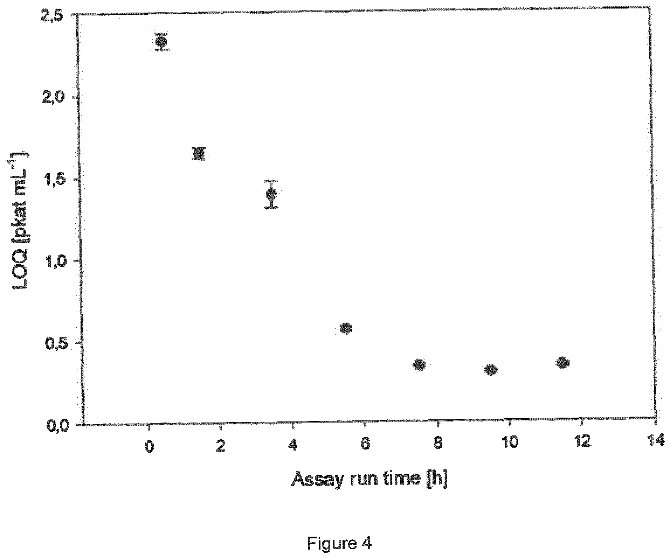

The absorption values of the reference samples without amylase were increasing with longer incubation times (data not shown). This is due to the instability of DA-67 and a kind of background activity caused by the glucose oxidase. This background activity is probably caused by sugars which are incorporated in the used starch which could serve as substrate for the glucose oxidase. The increase of the reference led to the increase of the LOD and the LOQ. To prevent this increase, the assay was performed in two steps: first the amylase was incubated in buffer which contains starch at 40 °C. After a certain incubation time, the other assay components were added and the increase of the absorption at 620 nm was measured kinetically. The sensitivity of the amylase assay was evaluated with run times between 0.5 and 11.5 h. The LOQ given as maltose concentration increased from 2.8 µM with 0.5 h run time to 9.6 µM with 11.5 h run time. The values for LOD showed a similar course, increasing from 1.4 µM to 5.9 µM with 0.5 to 11.5 h run time, respectively. That means that the longer the assay run time the higher the detectable/quantifiable maltose concentration. The LOQ values are given as volumetric activity and are shown in Figure 4.

The LOQ decreased from 2.3 pkat·mL⁻¹ with a run time of 0.5 h to 0.33 pkat·mL⁻¹ with a run time of 7.5 h. This corresponds to a concentration of 3.5 ng·mL⁻¹ of the commercial alpha-amylase and 3.2 ng·mL⁻¹ of the commercial maltogenic amylase. A run time of 7.5 h was set as maximum, as a further prolongation of the run time led to no further decrease of the LOQ.

The assay run time limitation to 7.5 h was valid for the direct determination of the activity of the commercial preparations. In later activity determinations it was possible to prolong the assay run time to 24 h as there another sample preparation was used, which includes a high dilution factor.

### Example 8:

### Sensitivity of the new assay in comparison to commercial assays

The sensitivity of the new amylase assay was compared to the sensitivity of the Ceralpha assay (determination of endo-amylase activities) and the Betamyl3 assay (determination of exo-amylase activities). The recommended incubation time of the Ceralpha and Betamyl3 assays was 20 min as written in the protocols from the supplier. Previous studies showed that the sensitivity of the Betamyl3 assay could be optimized by prolongation of the assay run time to 24 h and using a particular reference (reference 2). This particular reference contained the Betamyl3 reagent, the substrate, the ancillary enzyme, and buffer. The stopping solution was added to reference 2 at the end of the incubation time. This optimization principle was adopted in the current study. Different volumetric activities of the alpha-amylase (0.5 - 60 pkat·mL⁻¹) were used in the assay. Reference 1, which is given by the data sheet is prepared by the incubation of the Ceralpha reagent for a certain time followed by the addition of the stopping solution and sample/buffer solution. This reference 1 led to reliable results for short incubation times up to 3 h (Table 1). For longer incubation times, reference 1 led to an apparent increase of the activity. Reference 2, for which the Ceralpha reagent was incubated together with buffer followed by the addition of the stopping solution after the incubation time, allowed the prolongation of the assay run time to 24 h. It was possible to quantify a volumetric activity of 1 pkafi mL⁻' (Table 1). A further prolongation did not lead to a higher sensitivity. These findings are in correspondence to the results of the Betamyl3 assay, where it was also possible to determine 1 pkat·mL⁻¹ within 24 h.

The specific activities of the two tested amylase preparations and the LOQ values given as preparation concentrations for the original and optimized versions of the commercial assays and the new amylase assay are shown in Table 2. In addition, the increase of the sensitivity between the optimized versions of the commercial assays to the new amylase assay are given in Table 2.

It was possible to achieve a more than four times higher sensitivity with the new amylase assay compared to the optimized Ceralpha and Betamyl3 assay. This sensitivity was achieved after an assay run time of 7.5 h instead of 24 h. After an assay run time of about 3.5 h, the sensitivity of the optimized commercial assays was reached using the new amylase assay.

**Table 1: Determination of different endo-amylase activities using different blanks (B1 and B2).**

| Submitted endoamylase activity [pkat mL⁻¹] | Activity calculated with Blank 1^{*1} [pkat mL⁻¹] | Activity calculated with Blank 2^{*2} [pkat mL⁻¹] | Percentage activity with Blank 1 [%] | Percentage activity with Blank 2 [%] | Assay run time [h] |
|---|---|---|---|---|---|
| 60 | 60.91 ± 1.54 | 58.11 | 101.5 | 96.5 | 1.25 |
| 15 | 26.52 ± 0.98 | 15.35 | 176.8 | 102.3 | 2 |
| 10 | 18.20 ± 0.886 | 9.20 | 182.0 | 92.0 | 3 |
| 2 | 3.36 ± 0.074 | 1.94 | 168.0 | 97.0 | 17 |
| 1 | 3.73 ± 0.0303 | 0.93 | 373.0 | 93.0 | 24 |
| 0.5 | <LOD | <LOD | | | 48 |

| | | | | | |
|---|---|---|---|---|---|
| ^{*1} incubation of the Ceralpha reagent for the respective assay run time, addition of the stopping reagent followed by the addition of amylase solution ^{*2} incubation of the Ceralpha reagent together with heat inactivated amylase solution or buffer for the respective assay run time, addition of the stopping reagent | | | | | |

**Table 2: Comparison of the sensitivity of the original and optimized versions of the commercial assays and the new Da67 assay.**

| | **alpha-amylase (Ceralpha assay)** | **New assay** | | **Maltogenic amylase (Betamyl3 assay)** |
|---|---|---|---|---|
| | | **alpha-amylase** | **Maltogenic amylase** | |
| Enzyme activity [nkat mgₚᵣₑₚₐᵣₐₜᵢₒₙ⁻¹] | 67.9 | 96.9 | 104.4 | 83.3 |
| LOQ data sheet [ngpreparation mL⁻¹] | 25000 | n.s. | n.s. | 5724 |
| LOQ optimized [ngpreparation mL⁻¹] | 14.7 | 3.1 | 2.9 | 12.0 |
| Sensitivity improvement [-] | n.s. | 4.7 | 4.2 | n.s. |

| | | | | |
|---|---|---|---|---|
| n.s. not suitable | | | | |

Beside the higher sensitivity of the new assay also the usage of the natural substrate starch could be beneficial. The usage of the natural substrate allows the direct estimation of the amount of amylase needed for an industrial application. Using synthetic substrates, like the p-nitrophenylheptaoside in the Ceralpha assay or the p-nitrohphenyltrioside in the Betamyl3 assay do not allow such a direct estimation. To estimate the needed amount using a synthetic substrate a conversion factor has to be taken into account.

### Example 9:

### Determination of the residual activity of the α-amylase from B. subtilis in white bread

White bread was baked with three different dosages of the alpha-amylase from *B. subtilis* as recommended by the enzyme supplier. As reference, a bread without the addition of amylase was prepared under the same conditions. First, it was tried to determine a residual activity in the bread prepared with the highest amylase dosage using the optimized version of the Ceralpha assay. It was not possible to detect any residual activity with this assay. Then, the more sensitive new assay of the present invention was used for the determination of the potential residual activities in bread. The freeze-dried and ground bread crumb was directly used for activity determinations. The bread samples were suspended in buffer containing 0.01 % (w/v) soluble starch. A sample (to) was taken to determine the initial maltose content directly after resuspension. The bread samples were resuspended again and were incubated for 6 to 24 h at 40 °C dependent on the alpha-amylase dosage. For every incubation time also reference approaches using the bread without amylase addition were prepared. After the incubation time the bread particles were removed by centrifugation and the supernatant was used for the new amylase assay. The supernatant was diluted by the factor of 100 before using it in the assay because of residues from the bread extraction. Due to this dilution step it was possible to prolong the assay incubation time to more than 7.5 h. The residual activity of the alpha-amylase in bread was calculated using the differences of the sugar content between start and end of the incubation of the bread with amylase and the reference bread. The resulting activities for the tested dosages are shown in Table 3.

**Table 3: Alpha-amylase activities (B. subtilis) in white bread after baking using the new Da67 assay.**

| Dosage | | Residual amylase activity after baking | |
|---|---|---|---|
| Weight [*µg* g_{flour}⁻¹] | Activity [pkat g_{flour}⁻¹] | [pkat g_{flour}⁻¹] | [%] |
| 150 | 14538 | 300.5 | 2.07 ± 0.021 |
| 375 | 36345 | 830.3 | 2.28 ± 0.072 |
| 650 | 62998 | 1536.8 | 2.44 ± 0.109 |

The determined activity over the tested dosages was 2.26 ± 0.15 %. This corresponds to an overall standard deviation of 6.7 %. The residual activity of the alpha-amylase of *B. subtilis* is about 7.9 times lower than the residual activity of the maltogenic amylase from G. *stearothermophilus* which was previously determined to be 17.8 ± 1.24 %. In this previous study, also the temperature profile during baking of the bread was investigated. It was shown that the temperature was over 90 °C for about 20 min. The stability of the two amylases was also investigated in buffer at 90 °C. The residual activity of the alpha-amylase from *B. subtilis* after 10 min was 0.006 % and no activity was detectable after 20 min. The residual activity of the maltogenic amylase from G. *stearothermophilus* after 10 min was 1.7 %, 0.065 after 20 min and 0.037 % after 30 min. The residual activity of the maltogenic amylase in buffer was more than 300 times higher than the residual activity of the alpha-amylase. For both amylases the stability in dough seems to be significantly higher than in buffer. This stability enhancement could originate from matrix effects caused from the high substrate concentration and/or the low water activity. Previous studies investigated the stability of an amylase from *Bacillus sp.* WN11 in the presence from 5 to 20 % starch at 100 °C. An increase of the residual activity after 5 min incubation from 25 to 85 % was observed. This stabilizing effect of the dough might also be true for other thermostable enzymes used in the baking industry.

### Conclusion:

A new sensitive assay for the determination of endo- and exo-amylase activities using the natural substrate starch, glycogen, amylose, amylopectin, maltotriose, maltotetraose, or combinations thereof was developed. This new amylase assay is 4.7 times more sensitive than the optimized Ceralpha assay and 4.2 times more sensitive than the optimized Betamyl3 assay. The use of the natural substrate in comparison to synthetic substrates like in the Ceralpha and Betamyl3 assay allows the direct estimation of the amylase activity needed for an industrial application like for example in bread baking. The new amylase assay has the potential to be used in different industrial fields for the determination of endo- and exo-amylase activities in a low range of a minimum of 0.33 pkat·mL⁻¹.

For the first time it was possible to determine the residual activity of the alpha-amylase from *B. subtilis* in white bread using an activity-based assay. This determination could help in the discussion on the labeling enzymes on the bread package. However, the residual activities of the maltogenic amylase from G. *stearothermophilus* and the alpha-amylase from B. *subtilis* do not lead to a duty for its declaration on the package. Therefore, the residual activity has to have a functional property in the bread.

## Claims

1. A method for the detection of amylase activity in a sample, comprising the steps of
(a) adding a substrate to the sample, wherein said substrate is selected from the group consisting of starch, glycogen, amylose, amylopectin, maltotriose, maltotetraose, and combinations thereof,
(b) incubating the sample at conditions and for a time allowing for the enzymatic conversion of the substrate into glucose and maltose by amylases that might be present in the sample,
(c) adding to the sample incubated in step (b)
(i) glucose oxidase,
(ii) peroxidase, and
(iii) DA-67 (sodium;2-[[3,7-bis(dimethylamino)phenothiazine-10-carbonyl]amino]acetate),
(d) incubating the sample at conditions and for a time allowing for
(i) the enzymatic conversion of glucose and maltose that might have been generated in step (b) into oxidized glucose, oxidized maltose, and hydrogen peroxide (H₂O₂) by said glucose oxidase, and
(ii) the enzymatic conversion of DA-67 and H₂O₂ that might have been generated in step (d)(i) into reduced DA-67 (methylene blue; [7-(dimethylamino)phenothiazin-3-ylidene]-dimethyl-azanium;chloride) by said peroxidase, and
(e) detecting methylene blue in the sample.

2. The method of claim 1, wherein the amylase is selected from the group consisting of alpha-amylases, beta-amylases, maltogenic amylases, and alpha-glucosidases.

3. The method of claim 1 or claim 2, wherein the substrate is added to the sample in step (a) in the form of an aqueous solution.

4. The method of claim 3, wherein said aqueous substrate solution comprises the substrate in a concentration of from about 0.005 to about 0.015 % (w/v).

5. The method of any one of claims 1 to 4, wherein in step (c), glucose oxidase is added to the sample in an amount of about 10 to about 200 U·ml⁻¹ in the sample.

6. The method of any one of claims 1 to 5, wherein in step (c), peroxidase is added to the sample in an amount of about 1 to about 5 U·ml⁻¹ in the sample.

7. The method of any one of claims 1 to 6, wherein in step (c), DA-67 is added to the sample in an amount of about 15 to about 100 µM in the sample.

8. The method of any one of claims 1 to 7, wherein incubation in step (b) is incubation at about 37 to about 43 °C, for a duration of about 20 min to about 1.5 h.

9. The method of any one of claims 1 to 8, wherein incubation in step (d) is incubation at about 35 to about 40 °C, for a duration of about 0.5 to about 11.5 h.

10. The method of any one of claims 1 to 9, wherein the glucose oxidase is *Aspergillus niger* glucose oxidase.

11. The method of any one of claims 1 to 10, wherein the peroxidase is horseradish peroxidase.

12. The method of any one of claims 1 to 11, wherein amylase activity is detected in case methylene blue is detected in step (e).

13. The method of any one of claims 1 to 12, wherein detection in step (e) is quantitative detection, wherein step (e) comprises the steps of
(e1) detecting methylene blue in the sample,
(e2) comparing the amount of methylene blue detected in step (e1) to the amounts of methylene blue in a calibration curve correlating known amylase activities to said amounts of methylene blue, and
(e3) determining the amylase activity corresponding to the amount of methylene blue detected in step (e1) using said calibration curve;
or step (e) comprises the steps of
(e1') kinetically detecting methylene blue in the sample over time,
(e2') determining the slope of methylene detection over time, and
(e3') determining the difference in the slope determined in step (e2') to a reference, and
(e4') determining the amylase activity on the basis of the difference determined in step (e3').

14. The method of any one of claims 1 to 13, wherein detection in step (e) is photometric detection at a wavelength of 620 nm.

15. A kit, comprising
(a) a substrate, wherein said substrate is selected from the group consisting of starch, glycogen, amylose, amylopectin, maltotriose, maltotetraose, and combinations thereof,
(b) glucose oxidase,
(c) peroxidase, and
(d) DA-67 (sodium;2-[[3,7-bis(dimethylamino)phenothiazine-10-carbonyl]amino]acetate).
